(19) **Europäisches Patentamt European Patent Office Office européen des brevets**

(11) **EP 4 271 601 B1**

(12) **FASCICULE DE BREVET EUROPEEN**

(45) Date de publication et mention
de la délivrance du brevet:
**18.06.2025 Bulletin 2025/25**

(21) Numéro de dépôt: **21854682.8**

(22) Date de dépôt: **29.12.2021**

(51) Classification Internationale des Brevets (IPC):
***B60W 50/14*** *(2020.01)* ***B60W 40/08*** *(2012.01)*

(52) Classification Coopérative des Brevets (CPC):
**B60W 50/14; B60W 40/08;** B60W 2050/143;
B60W 2540/043; B60W 2540/221; B60W 2540/225

(86) Numéro de dépôt international:
**PCT/FR2021/052465**

(87) Numéro de publication internationale:
**WO 2022/144529 (07.07.2022 Gazette 2022/27)**

(54) **DISPOSITIF D'ANALYSE DE LA PERCEPTION D'UN DANGER PAR UN CONDUCTEUR DE VÉHICULE ET PROCÉDÉ ASSOCIÉ**

VORRICHTUNG ZUR ANALYSE DER WAHRNEHMUNG EINER GEFAHR EINES
FAHRZEUGFÜHRERS UND ZUGEHÖRIGES VERFAHREN

DEVICE FOR ANALYSING A VEHICLE DRIVER'S PERCEPTION OF A DANGER AND ASSOCIATED
METHOD

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.12.2020 FR 2014293**

(43) Date de publication de la demande:
**08.11.2023 Bulletin 2023/45**

(73) Titulaire: **Valeo Comfort and Driving Assistance
94000 Créteil (FR)**

(72) Inventeurs:
• **DEGRAND, Stephanie
94046 Creteil Cedex (FR)**
• **KERAUTRET, Laora
94046 Creteil Cedex (FR)**
• **NAVARRO, Jordan
69365 Lyon Cedex 7 (FR)**

(74) Mandataire: **Delplanque, Arnaud
Valeo Comfort and Driving Assistance
6 rue Daniel Costantini
94000 Créteil (FR)**

(56) Documents cités:
EP-A2- 1 182 089         DE-A1- 102011 109 564
DE-A1- 102016 201 939     DE-A1- 102019 004 822
DE-U1- 202016 008 400     US-A1- 2007 146 146

**Description**

La présente invention concerne le domaine technique de l'aide à la conduite dans un véhicule. Elle concerne plus précisément un dispositif d'analyse de la perception d'un danger par un conducteur de véhicule ainsi qu'un procédé associé.

A l'heure actuelle, il est difficile de savoir si un conducteur a perçu un danger présent dans son environnement. Le conducteur peut par exemple avoir regardé en direction d'un danger, sans avoir réalisé qu'il s'agissait là d'un danger, ou à l'inverse avoir perçu un danger en l'ayant seulement vu dans son champ de vision périphérique, mais sans l'avoir regardé directement. Il est encore possible que le conducteur n'ait pas du tout vu un danger, par exemple parce que ce dernier est hors de son champ de vision.

Le document EP1182089A2 porte sur un dispositif d'alerte d'un conducteur. Le document US2007146146A1 porte sur un dispositif évaluant le degré d'attention d'un conducteur en vue d'éviter une collision. Enfin, le document DE 10 2019 201 939 A1 décrit un dispositif d'amortissement de système de freinage.

On connaît déjà des dispositifs d'aide à la conduite embarqués dans les véhicules. De tels dispositifs d'aide à la conduite sont capables d'analyser l'environnement du véhicule dans lequel ils sont embarqués et d'intervenir auprès du conducteur, généralement sous la forme d'une alerte visuelle, sonore, haptique et/ou olfactive, de manière à le prévenir qu'un danger est susceptible de survenir dans son champ de conduite ou sur son trajet.

Les dispositifs d'aide à la conduite connus émettent de nombreuses alertes sans aucune garantie que lesdites alertes atteignent le conducteur. Or, ne pas savoir si un conducteur a correctement perçu un danger empêche de savoir si le conducteur se prépare à réagir convenablement face à ce danger, et empêche la conception de solutions de sécurité adaptées.

A l'inverse, lorsque les alertes du dispositif d'aide à la conduite atteignent le conducteur, ce dernier peut les considérer comme gênantes ou désagréables, notamment s'il a déjà perçu le danger associé à l'alerte, par exemple parce qu'il a déjà été prévenu de ce danger, ou parce qu'il a déjà vu et analysé de lui-même ce danger. Il arrive donc fréquemment que le conducteur préfère désactiver les alertes du dispositif d'aide à la conduite, quitte à courir le risque de ne pas être prévenu d'un danger qu'il n'aurait pas vu.

Il devient donc nécessaire de rendre les dispositifs d'aide à la conduite plus pertinents, afin qu'ils soient mieux acceptés par les conducteurs, par exemple en ne prévenant le conducteur que lorsque cela est réellement utile et en garantissant que l'alerte a bien atteint bien le conducteur.

Dans ce contexte, la présente invention propose un dispositif d'analyse de la perception d'un danger par un conducteur de véhicule, qui comprend :

- un capteur de fréquence cardiaque adapté à enregistrer la fréquence cardiaque du conducteur au cours du temps, et,
- une unité de traitement adaptée à :

  a) recevoir une indication du danger présent dans l'environnement du véhicule,
  b) analyser la fréquence cardiaque du conducteur mesurée par le capteur de fréquence, dans un intervalle de temps débutant à la réception de l'indication par l'unité de traitement, et
  c) déterminer si le danger a été perçu ou non par le conducteur selon que la fréquence cardiaque du conducteur analysée à l'étape b) comprend ou non une phase de décélération, dans ledit intervalle de temps.

Ainsi, le dispositif selon l'invention permet de déterminer si le conducteur est au courant d'un danger en analysant la fréquence cardiaque du conducteur immédiatement après que l'unité de traitement a été mise au courant du danger. Plus précisément, l'unité de traitement du dispositif selon l'invention considère que le danger a été perçu par le conducteur lorsque la fréquence cardiaque de ce dernier comprend une phase de décélération durant l'intervalle de temps d'analyse, par exemple dans les 3 secondes suivant la survenue du danger. La phase de décélération peut s'étendre sur tout l'intervalle de temps (c'est-à-dire ici sur 3 secondes ou plus), ou sur une partie seulement de l'intervalle de temps (c'est-à-dire ici sur une durée inférieure à 3 secondes). Des études ont montré qu'une telle phase de décélération cardiaque survient lorsque le cœur se met au ralenti pour que le cerveau puisse traiter une information qu'il a reçue. Selon la complexité de l'information que le cerveau a reçue et doit traiter, la durée de la phase de décélération cardiaque peut être plus ou moins longue, cette dernière étant au moins partiellement comprise dans un intervalle de temps d'une durée comprise entre 1 seconde et 5 secondes, notamment d'une durée de 3 secondes après la réception de l'information par le cerveau.

La détermination de la perception du danger par le conducteur grâce au dispositif selon l'invention est très fiable et rapide et n'engendre aucune perturbation ni gêne du conducteur.

D'autres caractéristiques non limitatives et avantageuses du dispositif conforme à l'invention, prises individuellement ou selon toutes les combinaisons techniquement possibles, sont les suivantes :

- à l'étape b), la fréquence cardiaque analysée est celle de l'intervalle de temps de mesure présentant une durée comprise entre 1 seconde et 5 secondes, de préférence entre 1 seconde et 3 secondes, à compter de la réception de l'indication ;

- le dispositif comprend un capteur oculaire adapté à détecter l'orientation du regard du conducteur, et l'unité de traitement détermine à l'étape c) que le danger a été perçu par le conducteur si la fréquence cardiaque du conducteur analysée à l'étape b) comprend une phase de décélération et si, en outre, le regard du conducteur est orienté dans le champ de conduite ;
- le dispositif comprend un capteur de la respiration du conducteur adapté à mesurer le rythme respiratoire du conducteur, et l'unité de traitement est adaptée à corriger la fréquence cardiaque du conducteur analysée à l'étape b) en fonction dudit rythme respiratoire mesuré ;
- l'unité de traitement détermine que la fréquence cardiaque du conducteur comprend une phase de décélération lorsque l'analyse de la fréquence cardiaque à l'étape b) montre que la variation de fréquence cardiaque du conducteur à un instant t présente une valeur inférieure à la moyenne des valeurs prises par la variation de fréquence cardiaque ($\Delta$FC) aux deux instants précédents l'instant t ;
- la variation de fréquence cardiaque du conducteur est calculée à partir d'une fréquence cardiaque de référence, choisie parmi : la fréquence cardiaque du conducteur mesurée par le capteur de fréquence cardiaque à l'instant où l'indication est reçue par l'unité de traitement à l'étape a) et une fréquence cardiaque moyenne du conducteur mesurée par le capteur de fréquence cardiaque , la fréquence cardiaque moyenne du conducteur étant de préférence mesurée en dehors de toute perception de danger.

Selon une caractéristique particulièrement avantageuse de l'invention, l'unité de traitement du dispositif selon l'invention est adaptée à mettre en œuvre une étape d) au cours de laquelle elle commande les interventions d'un dispositif d'aide à la conduite embarqué dans le véhicule auprès du conducteur, en fonction d'au moins un des éléments suivants : la perception ou non du danger par le conducteur déterminée à l'étape c), le moment de perception du danger par le conducteur, la durée de l'éventuelle phase de décélération de la fréquence cardiaque analysée à l'étape b), l'amplitude de l'éventuelle phase de décélération de la fréquence cardiaque analysée à l'étape b). Bien entendu, pour commander les interventions du dispositif d'aide à la conduite, l'unité de traitement 40 est adaptée à tenir compte, en sus d'au moins un des éléments précités, de l'indication du danger reçue à l'étape a), en particulier de la distance à laquelle se trouve le danger par rapport au véhicule, et de la dangerosité dudit danger.

Par le fait que l'unité de traitement « commande les interventions du dispositif d'aide à la conduite », on entend le fait qu'elle commande si ledit dispositif d'aide à la conduite doit intervenir auprès du conducteur ou non, quand il doit intervenir auprès du conducteur et comment (sous quelle forme) il doit intervenir auprès du conducteur. Notamment, le dispositif d'aide à la conduite intervient auprès du conducteur au sujet d'un danger si le conducteur n'est pas déjà au courant du danger, auquel cas l'intervention a lieu au moment le plus opportun, sous la forme la plus adéquate possible pour éviter le danger. Le dispositif selon l'invention est ainsi très utile au dispositif d'aide à la conduite embarqué dans le véhicule.

Les interventions du dispositif d'aide à la conduite auprès du conducteur comprennent, au choix, l'émission d'un signal de la part du dispositif d'aide à la conduite à destination du conducteur, le signal pouvant prendre la forme d'une alerte visant à prévenir le conducteur d'un danger ou la forme d'un conseil visant à guider le conducteur face au danger, ou la prise en main forcée du véhicule par le dispositif d'aide à la conduite, par exemple en imposant au conducteur un passage à un mode de conduite autonome du véhicule, ou un passage à une conduite du véhicule par téléassistance.

Par exemple, si le dispositif selon l'invention détermine que le conducteur n'a pas perçu un danger, l'unité de traitement commande que le dispositif d'aide à la conduite doit intervenir auprès du conducteur, et commande si l'intervention doit prendre la forme d'une alerte émise pour prévenir le conducteur de ce danger, et/ou de conseils émis pour guider le conducteur face au danger et/ou la forme d'une aide à la conduite imposée au conducteur. Lorsque l'aide à la conduite est imposée au conducteur par le dispositif d'aide à la conduite, on considère que le dispositif d'aide à la conduite prend en main le véhicule dans la mesure où les organes du véhicule sont alors directement actionnés par le dispositif d'aide à la conduite pour éviter le danger. A l'inverse, si le dispositif selon l'invention détermine que le conducteur est déjà au courant d'un danger, le dispositif d'aide à la conduite met les alertes associées à ce danger sous silence, de sorte que le conducteur n'est pas gêné par l'émission d'alertes inutiles.

D'autres caractéristiques non limitatives et avantageuses du dispositif conforme à l'invention, prises individuellement ou selon toutes les combinaisons techniquement possibles, sont les suivantes :

- le dispositif selon l'invention comprend le dispositif d'aide à la conduite, et l'indication du danger reçue par ladite unité de traitement à l'étape a) est émise par ledit dispositif d'aide à la conduite ;
- l'unité de traitement est en outre adaptée à identifier le conducteur et à personnaliser, en fonction de ladite identification l'analyse de la fréquence cardiaque mise en œuvre à l'étape b);,
- l'unité de traitement est en outre adaptée à personnaliser la commande de l'étape d) en fonction de ladite identification.
- l'unité de traitement est en outre adaptée à personnaliser la commande de l'étape d) en fonction de ladite identification.

L'invention concerne enfin un procédé d'analyse de la perception d'un danger par un conducteur de véhicule, selon lequel il est prévu de mettre en œuvre les étapes de :

- mesure de la fréquence cardiaque du conducteur,
- réception d'une indication du danger présent dans l'environnement du véhicule,
- analyse de la fréquence cardiaque du conducteur mesurée, dans un intervalle de temps débutant à la réception de l'indication du danger, et
- détermination de la perception ou non du danger par le conducteur selon que la fréquence cardiaque du conducteur analysée comprend ou non une phase de décélération dans ledit intervalle de temps.

De plus, diverses autres caractéristiques de l'invention ressortent de la description annexée effectuée en référence aux dessins qui illustrent des formes, non limitatives, de réalisation de l'invention et où :

[Fig. 1] est une représentation schématique d'un dispositif d'analyse de la perception d'un danger, conforme à l'invention,

[Fig. 2] est un graphique représentant deux courbes FC1 et FC2 donnant l'évolution de la variation de fréquence cardiaque ΔFC (en battements par minute, ou bpm) au cours du temps (en secondes ou s), pour un même conducteur, dans deux situations de conduite différentes,

[Fig. 3] est un graphique représentant deux courbes V1 et V2 donnant l'évolution de la variation de vitesse du véhicule (en kilomètres par heure ou km/h) au cours du temps (en secondes ou s) pour le conducteur dans les deux situations de la figure 2, et

[Fig. 4] est une représentation schématique des étapes principales d'un procédé selon l'invention.

Il est à noter que sur ces figures les éléments structurels et/ou fonctionnels communs aux différentes variantes peuvent présenter les mêmes références.

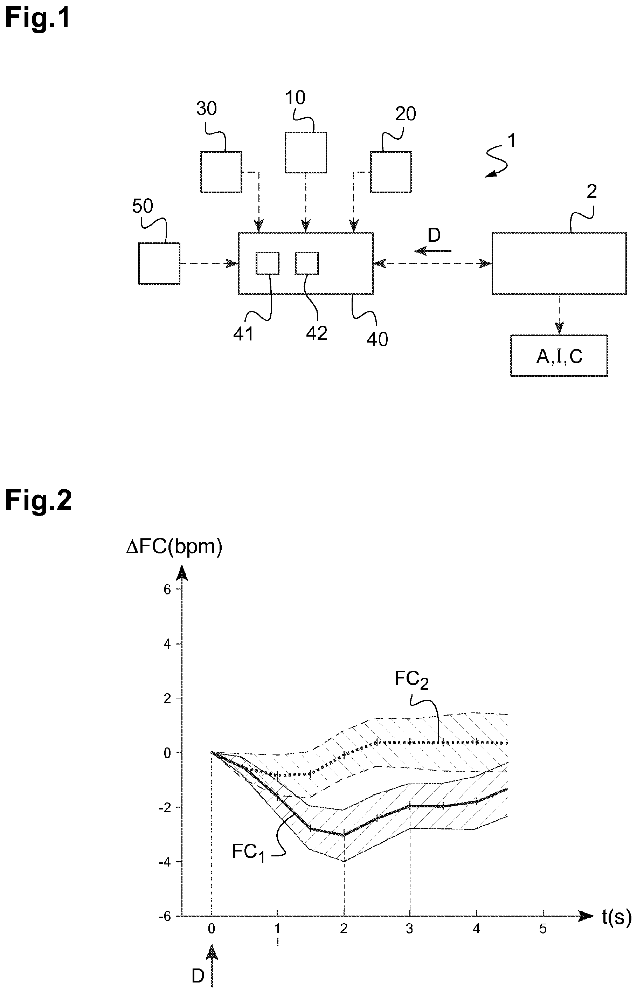

Sur la figure 1, on a représenté schématiquement les éléments principaux d'un dispositif 1 d'analyse de la perception d'un danger par un conducteur de véhicule. Le dispositif 1 est particulièrement adapté à l'analyse de la perception d'un danger dans un véhicule automobile. La suite de la description détaillera ce cas de figure. Il est cependant tout à fait envisageable que le dispositif 1 selon l'invention soit utilisé pour l'analyse de la perception d'un danger dans un autre type de véhicule, par exemple un bateau, un avion ou encore un train.
Le dispositif 1 est ici embarqué dans le véhicule automobile. Il est cependant tout à fait envisageable qu'une partie au moins des éléments qu'il comprend soit située à distance du véhicule.
Le dispositif 1 comprend au moins:

- un capteur de fréquence cardiaque 10 adapté à mesurer la fréquence cardiaque du conducteur au cours du temps, et,
- une unité de traitement 40 adaptée à :

a) recevoir une indication du danger présent dans l'environnement du véhicule,
b) analyser la fréquence cardiaque du conducteur mesurée par le capteur de fréquence cardiaque 10, dans un intervalle de temps débutant à la réception de l'indication par l'unité de traitement 40 à l'étape a), et
c) déterminer si le danger a été perçu ou non par le conducteur selon que la fréquence cardiaque du conducteur analysée à l'étape b) comprend ou non une phase de décélération dans ledit intervalle de temps.

Le capteur de fréquence cardiaque 10 du conducteur est embarqué dans le véhicule. Il comprend un capteur de l'activité électrique du cœur et/ou un capteur de l'activité mécanique du cœur.
Selon un premier exemple, le capteur de fréquence cardiaque 10 comporte un capteur d'activité électrique sous la forme d'une ou plusieurs électrodes, placées directement au contact de la peau du conducteur, pour enregistrer le signal électrique engendrée par l'activité cardiaque. Dans ce premier exemple, le capteur de fréquence cardiaque 10 est par exemple placé sur le volant, à l'endroit où le conducteur place ses mains sur le volant.
Selon un autre exemple, le capteur de fréquence cardiaque 10 comporte un capteur d'activité mécanique sous la forme d'une caméra à afflux sanguin ou d'un radar capable d'enregistrer le signal balistique de l'activité cardiaque du conducteur, le signal balistique étant directement lié à l'activité mécanique générée par les battements cardiaques. Dans ce deuxième exemple, le capteur de fréquence cardiaque 10 mesure la fréquence cardiaque à travers les vêtements du conducteur, et sans avoir besoin d'être en contact avec ledit conducteur. Le capteur de fréquence cardiaque 10 peut par exemple être placé dans le siège du conducteur, derrière le volant (c'est-à-dire entre le volant et le parebrise du véhicule), dans la ceinture du conducteur, ou encore une combinaison de ses endroits.
Selon un troisième exemple, le capteur de fréquence cardiaque 10 comporte à la fois un capteur d'activité électrique et un capteur d'activité mécanique (ici balistique), par exemple disposés sur un ou plusieurs des endroits cités précédemment.
Quel que soit l'exemple envisagé, le capteur de fréquence cardiaque 10 fonctionne en continu pour enregistrer la fréquence cardiaque FC du conducteur au cours du temps, pendant les trajets du conducteur. Il mesure la fréquence cardiaque instantanée, c'est-à-dire le nombre de battements cardiaques effectués en une minute (donnée en battements par minute ou bpm). Le capteur de fréquence cardiaque 10 donne ici la fréquence cardiaque instantanée FC(t) du conducteur à intervalle de temps régulier, par exemple toutes les 0,5 secondes (s). Bien entendu, il peut donner la fréquence cardiaque instantanée à des intervalles de temps plus courts, et éventuellement irréguliers. Avant de pouvoir donner sa première mesure, il est préférable que le capteur de fréquence cardiaque 10 ait fonctionné pendant au moins une minute.

En pratique, pour pouvoir donner la fréquence cardiaque instantanée du conducteur, le capteur de fréquence cardiaque 10 met en œuvre les étapes suivantes :

- il récupère la durée écoulée entre deux battements cardiaques (ou paire de battement cardiaque) provenant du conducteur, et ce pour une pluralité de paires de battement cardiaque,
- à partir desdites durées et du nombre de battements cardiaques mesurés, il établit une fréquence cardiaque pour le conducteur, et,
- il effectue une interpolation pour obtenir la fréquence cardiaque du conducteur toutes les 0,5 secondes.

L'unité de traitement 40 est quant à elle adaptée à communiquer avec le capteur de fréquence 10. Ici, elle comporte une mémoire 41 pour enregistrer, en fonction du temps, la fréquence cardiaque du conducteur mesurée par le capteur de fréquence cardiaque 10. Plus précisément, la mémoire 41 enregistre la fréquence cardiaque instantanée FC(t) du conducteur à chacune des mesures du capteur de fréquence cardiaque 10, à savoir dans cet exemple toutes les 0,5 secondes (s).

A l'étape a), l'unité de traitement 40 reçoit l'indication selon laquelle un danger est présent dans l'environnement du véhicule. Cette indication provient d'un dispositif d'aide à la conduite 2 embarqué dans le véhicule automobile avec lequel l'unité de traitement 40 est adaptée à communiquer. On considère ici que le dispositif d'aide à la conduite 2 est compris dans le dispositif 1 selon l'invention. Il est cependant tout à fait envisageable que le dispositif d'aide à la conduite soit distinct du dispositif 1 selon l'invention, tout en étant adapté à communiquer avec l'unité de traitement 40 du dispositif 1.

Le dispositif d'aide à la conduite 2 est connu en soi et ne sera pas décrit dans le détail. Pour l'essentiel, le dispositif d'aide à la conduite 2 comprend une pluralité de capteurs disposés dans le véhicule et/ou à l'extérieur de ce dernier de manière qu'il est capable de sonder l'environnement plus ou moins lointain du véhicule pour déterminer des situations dangereuses (ou dangers) dans cet environnement, par exemple un piéton susceptible de traverser devant le véhicule, un véhicule roulant à contre sens, un freinage brutal d'un autre véhicule situé devant le véhicule, un accident devant le véhicule, etc.

Le danger détecté par le dispositif d'aide à la conduite 2 est ici défini en fonction de sa gravité et/ou de sa distance par rapport au véhicule. L'indication D comprend donc une information sur la gravité (ou dangerosité) et/ou la distance du danger par rapport au véhicule.

Le dispositif d'aide à la conduite 2 est en particulier adapté à émettre à destination de l'unité de traitement 40, l'indication D selon laquelle il a déterminé qu'un danger était présent dans l'environnement du véhicule, et ce dès qu'il a déterminé ledit danger. Lorsque le dispositif d'aide à la conduite émet une telle indication D à destination de l'unité de traitement 40 (étape a), l'unité de traitement 40 détermine que l'instant auquel elle a reçu l'indication D de la part du dispositif d'aide à la conduite 2 correspond à l'instant initial, ou l'instant t=0s, pour l'analyse de l'étape b).

Comme le montre la figure 1, le dispositif d'aide à la conduite 2 est aussi capable d'intervenir auprès du conducteur, notamment en émettant à destination du conducteur une alerte A pour prévenir le conducteur du danger. Cette alerte A peut être émise selon différentes modalités : sonore (par exemple un bip ou une annonce orale), visuelle (par exemple un message s'affichant sur le tableau de bord), haptique (par exemple une vibration ressentie dans le volant ou dans le siège), olfactive (par exemple une odeur de brûlé diffusée dans l'habitacle), ou encore selon une combinaison de ces différentes modalités.

A l'étape b), l'unité de traitement 40 analyse la fréquence cardiaque du conducteur mesurée par le capteur de fréquence cardiaque 10, dans un intervalle de temps débutant à l'instant initial (t=0s) de réception de l'indication D. De façon préférentielle, la fréquence cardiaque FC analysée à l'étape b) est celle mesurée dans l'intervalle de temps présentant une durée comprise entre 1 seconde et 5 secondes, encore plus préférentiellement pendant une durée comprise entre 1 seconde et 3 secondes, à compter de la réception de l'indication D. Par exemple, l'intervalle de temps débute à la réception de l'indication D de danger et s'étend jusqu'à 3 secondes après ladite réception de l'indication D.

Pour effectuer l'analyse, l'unité de traitement 40 comprend ici un calculateur 42 adapté à exécuter ladite analyse de fréquence cardiaque. Cette analyse comprend notamment le calcul de la variation de fréquence cardiaque ΔFC(t) du conducteur à instant donné, selon la formule suivante :

$$\Delta FC(t) = FC(t) - FCréf$$

où FC(t) est la fréquence cardiaque instantanée du conducteur, mesurée par le capteur de fréquence cardiaque 10 à l'instant t, et FCréf est une fréquence cardiaque de référence associée au conducteur.

Ici, la fréquence cardiaque de référence FCréf associée au conducteur est choisie parmi la liste suivante :

- la fréquence cardiaque instantanée FC(t=0s) du conducteur mesurée par le capteur de fréquence cardiaque 10 à l'instant t=0s où l'indication D est reçue par l'unité de traitement 40 à l'étape a), et

- une fréquence cardiaque moyenne du conducteur.

De préférence, la fréquence cardiaque de référence FCréf associée au conducteur est choisie comme la fréquence cardiaque instantanée FC(t=0s) du conducteur mesurée par le capteur de fréquence cardiaque 10 à l'instant t=0s.

Lorsqu'elle est choisie comme la fréquence cardiaque moyenne du conducteur, on considère que la fréquence cardiaque de référence FCréf est une fréquence moyenne spécifique du conducteur calculée par le calculateur 42 à partir de la mesure de la fréquence cardiaque du conducteur sur une période d'au moins 1 minute, de préférence en dehors de tout danger dans l'environnement. La fréquence cardiaque moyenne du conducteur est alors de préférence recalculée et réenregistrée régulièrement dans la mémoire 41 de l'unité de traitement 10. Il est aussi tout à fait envisageable de considérer que la fréquence cardiaque moyenne du conducteur est la fréquence cardiaque du conducteur au repos. Dans ce cas, la fréquence cardiaque moyenne est issue d'une phase d'initialisation du dispositif 1 selon l'invention, au cours de laquelle la fréquence cardiaque du conducteur est enregistrée, véhicule à l'arrêt.

En pratique, le calculateur 42 calcule la valeur ΔFC(t) prise par la variation de fréquence cardiaque à chacun des instants t postérieurs à l'instant initial (t=0s) auquel le capteur de fréquence cardiaque 10 a mesuré la fréquence cardiaque instantanée FC(t).

A partir des valeurs calculées, on peut par exemple représenter graphiquement la courbe donnant l'évolution de la variation de fréquence cardiaque ΔFC au cours du temps. Sur la figure 2, on a par exemple représenté graphiquement deux courbes FC1 et FC2 qui représentent l'évolution de la variation de fréquence cardiaque ΔFC au cours du temps, pour un même conducteur, dans deux situations différentes. Chaque courbe FC1 et FC2 est ici entourée d'une zone représentant l'erreur associée à la valeur représentée à l'instant t. La zone d'erreur est représentée en hachuré pour chaque courbe FC1 et FC2. Dans la première situation, associée à la courbe FC1, l'unité de traitement 40 a reçue, à l'instant initial (t=0s), symbolisé par la flèche sur la figure 2, l'indication D d'un véhicule roulant à contresens sur le trajet du véhicule conduit par le conducteur. Dans la deuxième situation, associée à la courbe FC2, l'unité de traitement 40 a reçu à l'instant initial (t=0s) une indication D d'un danger susceptible de survenir sur le trajet du véhicule conduit par le conducteur.

A l'étape c), l'unité de traitement 40 détermine enfin si le conducteur a perçu ou non le danger, en fonction de l'analyse de l'étape b).

Plus précisément, si la fréquence cardiaque du conducteur analysée à l'étape b) comprend une phase de décélération (aussi appelée « décélération cardiaque »), dans ledit intervalle de temps de mesure, de préférence dans l'intervalle de temps [0 ; 3s] d'une durée de 3 secondes, alors l'unité de traitement 40 détermine que le conducteur a perçu le danger. Au contraire, si la fréquence cardiaque du conducteur analysée à l'étape b) ne comprend aucune phase de décélération cardiaque, dans ledit intervalle de temps de mesure préféré [0 ; 3s], alors l'unité de traitement 40 détermine que le conducteur n'a pas perçu le danger.

Pour déterminer si la fréquence cardiaque comprend une phase de décélération cardiaque, l'unité de traitement 40 regarde si la variation de fréquence cardiaque ΔFC du conducteur issue de l'analyse de l'étape b) comprend une phase de décroissance au cours du temps. Plus particulièrement, le calculateur 42 compare les valeurs successives prises par la variation de fréquence cardiaque ΔFC, dans l'intervalle de temps de mesure, par exemple compris entre 0s et 3s. L'unité de traitement 40 détermine que la variation de fréquence cardiaque ΔFC du conducteur comprend une phase de décroissance lorsque la variation de fréquence cardiaque à un instant t ΔFC(t) présente une valeur inférieure à la moyenne arithmétique des valeurs prises par ladite variation de fréquence cardiaque aux deux instants précédents l'instant t. Autrement dit, le calculateur 42 calcule, pour chaque instant de mesure t, la moyenne arithmétique MOY des deux valeurs prises par la variation de fréquence cardiaque aux deux instants (t-1) et (t-2) précédents l'instant t selon la formule suivante :

$$MOY = \frac{\Delta FC(t-1) + \Delta FC(t-2)}{2}$$

Le calculateur 42 compare ensuite la valeur prise par la variation de fréquence cardiaque à l'instant t ΔFC(t), avec ladite moyenne MOY. Si la variation de fréquence cardiaque à l'instant t est inférieure à la moyenne, soit ΔFC(t) < MOY, alors l'unité de traitement 40 détermine que la fréquence cardiaque comprend une phase de décélération sur les trois instants t-2, t-1 et t.

Ainsi, sur l'exemple de la figure 2, l'unité de traitement 40 détermine que la courbe FC1 donnant l'évolution de la variation de fréquence cardiaque au cours du temps, comprend une phase de décroissance, dans l'intervalle s'étendant ici entre 0 et 2 secondes après la réception de l'indication D par l'unité de traitement 40. L'unité de traitement 40 conclut alors que dans la première situation, le conducteur a perçu le danger que représentait le véhicule en contresens, dans les deux secondes suivant la réception de l'indication D par l'unité de traitement 40. La perception par le conducteur peut être liée au fait que le conducteur a vu lui-même le véhicule en contresens au loin, ou au fait que le dispositif d'aide à la conduite 2 a prévenu le conducteur au moyen d'une alerte A, en même temps qu'il a envoyé l'indication D à l'unité de traitement 40.

Au contraire, sur la figure 2, la courbe FC2 ne comprend pas de phase de décroissance dans l'intervalle de mesure s'étendant entre 0 et 5 secondes après la réception de l'indication D par l'unité de traitement 40. L'unité de traitement 40 conclut que le conducteur dans la deuxième situation n'a pas perçu de danger. Il est notamment possible que le conducteur n'ait pas vu de lui-même le danger dans cette deuxième situation, ou qu'il n'ait pas perçu l'éventuelle alerte A émise par le dispositif d'aide à la conduite 2, ou encore que le dispositif d'aide à la conduite 2 ait été défaillant pour émettre l'alerte A.

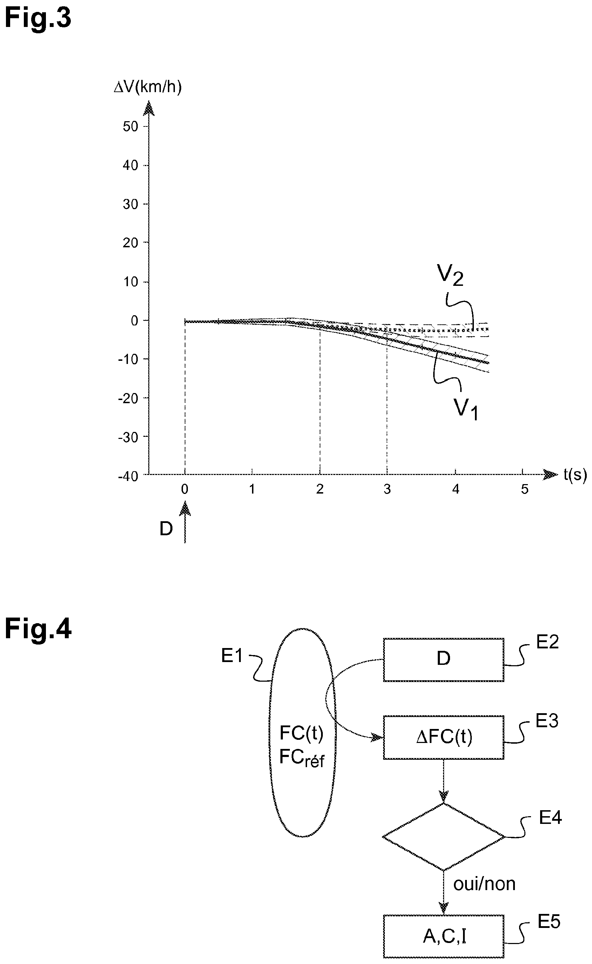

Sur la figure 3, on a représenté graphiquement deux courbes V1 et V2 qui représentent l'évolution de la variation de vitesse ΔV au cours du temps, pour le véhicule conduit par le conducteur dans les deux situations décrites précédemment. Les courbes V1 et V2 sont ici chacune entourées d'une zone représentant l'erreur associée à la valeur représentée à l'instant t. La zone d'erreur est ici représentée en hachuré pour chaque courbe V1 et V2. La variation de vitesse du véhicule est calculée par la formule suivante :

$$\Delta V(t) = V(t) - Vréf$$

où V(t) est la vitesse instantanée du conducteur à l'instant t, et Vréf est la vitesse de référence du véhicule, ici choisie comme la vitesse instantanée du véhicule à l'instant initial (t=0s).

Les conclusions de l'unité de traitement 40 du dispositif 1 selon l'invention, quant à la perception du danger par le conducteur, sont confirmées par les réactions différentes du conducteur, visibles sur la figure 3, dans les deux situations. En effet, après avoir perçu le danger dans la première situation, le conducteur ralentit le véhicule qu'il conduit pour être prudent face au danger, de sorte que la courbe V1 présente une décroissance après les 2 secondes qu'il a fallu au conducteur pour analyser l'information du danger que son cerveau avait reçu. Au contraire, la courbe V2 montre une variation de vitesse nulle au cours du temps, ce qui signifie que le conducteur a maintenu sa vitesse constante dans la deuxième situation. Cela confirme que le conducteur n'a pas perçu de danger dans cette deuxième situation.

Le dispositif 1 décrit précédemment peut comprendre divers perfectionnements avantageux, tout à fait cumulables les uns avec les autres.

Selon un premier perfectionnement avantageux envisageable, le dispositif 1 est capable de mesurer avec une précision accrue la fréquence cardiaque du conducteur.

Le dispositif 1 selon le premier perfectionnement comprend à cet effet un capteur de la respiration 20 du conducteur, adapté à mesurer le rythme respiratoire du conducteur au cours du temps. Le capteur de respiration 20 est par exemple placé en face du siège du conducteur, derrière le volant. Il est envisageable que le capteur de respiration soit confondu avec le capteur de fréquence cardiaque 10, notamment lorsque le capteur de fréquence cardiaque 10 comporte un capteur d'activité mécanique du cœur. Le capteur de respiration 20 est synchronisé avec le capteur de fréquence cardiaque 10 de manière à mesurer le rythme respiratoire du conducteur aux mêmes instants auxquels est mesurée la fréquence cardiaque instantanée du conducteur. Le capteur de respiration 20 est adapté à mesurer la fréquence respiratoire du conducteur, c'est-à-dire le nombre de cycle « inspiration + expiration » effectué par le conducteur dans un intervalle de temps d'une minute. Comme le capteur de fréquence cardiaque 10, le capteur de rythme respiratoire 20 (ou capteur de fréquence respiratoire) doit de préférence avoir fonctionné pendant au moins une minute avant de donner sa première mesure.

Selon ce premier perfectionnement, l'unité de traitement 40 est adaptée à communiquer avec le capteur de respiration 20 pour enregistrer dans la mémoire 41 le rythme respiratoire du conducteur en fonction du temps. Plus précisément, la mémoire 41 enregistre le rythme respiratoire du conducteur à chacune des mesures du capteur de respiration 20, à savoir dans cet exemple toutes les 0,5 secondes (s).

L'unité de traitement 40, grâce à son calculateur 42, est alors adaptée à corriger la fréquence cardiaque instantanée FC(t) du conducteur en fonction dudit rythme respiratoire mesuré. La correction consiste à éliminer du signal de fréquence cardiaque mesuré par le capteur de fréquence 10, le signal de rythme respiratoire mesuré par le capteur de respiration 20, de sorte que la fréquence cardiaque instantanée du conducteur ne soit plus influencée par les inspirations et expirations du conducteur qui font naturellement osciller ladite fréquence cardiaque.

Selon un deuxième perfectionnement avantageux, le dispositif 1 selon l'invention comprend un moyen pour éviter de détecter des faux-positifs, c'est-à-dire éviter de déterminer que le conducteur a perçu un danger alors que ce n'est pas le cas.

Plus précisément, le dispositif 1 selon l'invention comprend un capteur oculaire 30 adapté à détecter l'orientation du regard du conducteur. Le capteur oculaire 30 prend par exemple la forme d'une caméra, placée derrière le volant. Le capteur oculaire 30 est adapté à déterminer si le regard du conducteur est orienté en direction du champ de conduite, ou s'il est orienté en direction d'un élément associé à la conduite, ou encore s'il est orienté en direction d'un élément annexe, qui détourne le conducteur de la conduite. On entend ici par « champ de conduite » la route visible par le conducteur lorsque ce dernier conduit le véhicule. Les « éléments associés à la conduite » comprennent par exemple : les rétroviseurs, le système de navigation ou encore le tableau de bord d'où peuvent provenir des informations sur le véhicule (vitesse, voyants qui s'allument...). On considère que les éléments annexes comprennent tous les autres éléments contenus dans le véhicule qui ne participent pas à la conduite, par exemple le téléphone portable du conducteur, le poste de radio, les passagers du véhicule. Par exemple, le capteur oculaire 30 est adapté à détecter si le conducteur a la tête baissée pour regarder son téléphone portable ou s'il est retourné pour observer les passagers de la banquette arrière, ou le passager assis à côté de lui, auquel cas son regard n'est ni orienté vers le champ de conduite, ni vers un élément associé à la conduite.

Le capteur oculaire 30 permet à l'unité de traitement 40 d'utiliser un critère supplémentaire pour déterminer si le conducteur a perçu ou non le danger : la direction du regard du conducteur. Ce critère supplémentaire évite à l'unité de traitement 40 de déterminer que le conducteur a perçu un danger uniquement car sa fréquence cardiaque comporte une phase de décélération cardiaque dans l'intervalle de temps observé. En effet, la décélération cardiaque du conducteur n'est pas forcément due à la perception d'un danger dans le champ de conduite mais peut être due à un autre évènement, comme à la lecture d'un sms par exemple, ou à des voyants inquiétants s'allumant sur le tableau de bord. Ainsi, grâce au capteur oculaire 30, l'unité de traitement 40 utilise deux critères pour déterminer que le danger a été perçu par le conducteur : le fait que sa fréquence cardiaque comprend une phase de décélération et le fait que son regard est orienté dans le champ de conduite. Autrement dit, l'unité de traitement 40 détermine à l'étape c) que le danger a été perçu par le conducteur, uniquement si la fréquence cardiaque du conducteur analysée à l'étape b) comprend une phase de décélération et si, en outre, le regard du conducteur est orienté dans le champ de conduite. En revanche, dès lors que la fréquence cardiaque du conducteur analysée à l'étape b) ne comporte pas de phase de décélération, l'unité de traitement 40 conclut directement que le danger n'a pas été perçu, sans avoir à regarder la direction du regard du conducteur.

Ainsi, selon ce deuxième perfectionnement avantageux, le dispositif selon l'invention gagne en précision dans la détermination de la perception du danger par le conducteur, en permettant d'élucider la cause de la modulation de la fréquence cardiaque, ladite cause, pouvant venir par exemple de la route, ou de l'habitacle (voyant suspect allumé) ou encore d'un texto reçu sur le portable du conducteur.

Selon un troisième perfectionnement avantageux, l'unité de traitement 40 du dispositif 1 est adaptée à commander le dispositif d'aide à la conduite 2, en particulier commander s'il doit intervenir auprès du conducteur, quand il doit intervenir auprès du conducteur et comment il doit intervenir auprès du conducteur. Grâce à cette commande, le dispositif d'aide à la conduite 2 perturbe moins le conducteur lorsqu'il intervient auprès de lui, et les éventuelles interventions du dispositif d'aide à la conduite 2 à destination du conducteur sont plus pertinentes car réalisées au moment opportun, sous une forme appropriée.

Comme il a été dit précédemment, le dispositif d'aide à la conduite 2 est ici capable d'intervenir auprès du conducteur, par exemple en émettant des alertes A (de modalités sonore, visuelle, haptique et/ou olfactives) à destination du conducteur afin de le prévenir d'un danger que ledit dispositif d'aide à la conduite aurait repéré dans l'environnement du véhicule. Les modalités d'émission de chaque alerte A peuvent être combinées ou non. L'émission d'une alerte A peut être ponctuelle ou répétée dans le temps, par exemple à intervalle de temps régulier.

Ici, le dispositif d'aide à la conduite 2 est aussi capable d'intervenir auprès du conducteur en émettant à destination du conducteur des conseils C pour l'aider à réagir face à un danger. De tels conseils C émis par le dispositif d'aide à la conduite 2 sont par exemple les suivants : demander au conducteur de ralentir, de se rabattre sur une voie précise de circulation, de se mettre sur le bas-côté, etc. Les conseils C qui sont destinés à guider le conducteur pour qu'il réagisse au mieux face à un danger sont généralement donnés selon une modalité d'émission visuelle (affichage sur le tableau de bord) ou sonore (conseils vocaux émis dans l'habitacle). Les modalités d'émission de chaque conseils C peuvent être combinées ou non. L'émission d'un conseil C peut être ponctuelle ou répétée au cours du temps, par exemple à intervalle de temps régulier.

Le dispositif d'aide à la conduite 2 est aussi capable, ici, d'intervenir auprès du conducteur en lui imposant une aide à la conduite I. Une telle aide à la conduite I peut consister à décharger le conducteur de la conduite, par exemple en lui imposant une conduite à distance par téléassistance ou en lui imposant un passage à un mode de conduite autonome du véhicule. Ainsi, les aides à la conduite imposées par le dispositif de conduite 2 reviennent à prendre la main sur les organes du véhicule et à les actionner à la place du conducteur.

Dans ce contexte, l'unité de traitement 40 du dispositif 1 selon l'invention est adaptée à mettre en œuvre une étape d) au cours de laquelle elle commande les interventions du dispositif d'aide à la conduite 2 auprès du conducteur. En particulier, l'unité de traitement 40 commande si le dispositif d'aide à la conduite 2 doit ou non émettre ou réémettre une alerte A à destination du conducteur pour le prévenir d'un danger, à quel moment, sous quelle modalité et à quelle fréquence, s'il doit ou non émettre un ou des conseils C de conduite pour guider le conducteur face au danger, à quel moment, sous quelle modalité et à quelle fréquence, et/ou s'il doit ou non imposer une aide à la conduite I au conducteur pour éviter le danger et à quel moment.

Plus précisément, l'unité de traitement 40 met en œuvre la commande de l'étape d) en fonction d'au moins un des éléments suivants : la perception ou non du danger par le conducteur déterminée à l'étape c), le moment de perception du danger par le conducteur, la durée de l'éventuelle phase de décélération de la fréquence cardiaque analysée à l'étape b), l'amplitude de l'éventuelle phase de décélération de la fréquence cardiaque analysée à l'étape b).

L'analyse de la fréquence cardiaque à l'étape b) permet à l'unité de traitement 40 de savoir non seulement si le conducteur a perçu le danger ou non, mais encore s'il l'a perçu précocement, c'est-à-dire si la décélération cardiaque s'est terminée tout au début de l'intervalle d'analyse, par exemple sur les deux premières secondes de l'intervalle [0-3s], ou tardivement, c'est-à-dire si la décélération cardiaque a débuté tout à la fin de l'intervalle d'analyse, par exemple sur la dernière demi-seconde de l'intervalle [0-3s]. Grâce à l'analyse de la fréquence cardiaque à l'étape b), l'unité de traitement 40 peut donc déterminer quel est le moment le plus opportun pour que le dispositif d'aide à la conduite 2 interagisse avec le conducteur. Par exemple, le moment le plus opportun est déterminé par l'unité de traitement 40 en fonction de la vitesse de décroissance de la variation de fréquence cardiaque dans la phase de décroissance. Plus précisément, si la vitesse de décroissance est supérieure à une valeur seuil prédéterminée (c'est-à-dire que la vitesse de décroissance est rapide), l'unité de traitement 40 commande au dispositif d'aide à la conduite 2 de ne pas intervenir, ou de mettre ses alertes sous silence. Au contraire, si la vitesse de décroissance est inférieure à ladite valeur seuil prédéterminée (c'est-à-dire que la vitesse de décroissance est lente), l'unité de traitement 40 commande au dispositif d'aide à la conduite 2 d'intervenir pour alerter et/ou conseiller le conducteur, voire pour intervenir sur la conduite. En pratique, si l'unité de traitement 40 ne décèle aucune décélération cardiaque sur l'intervalle de temps d'analyse, ici [0 ; 3s] après la réception de l'indication D de danger, elle commande au dispositif d'aide à la conduite 2 d'intervenir auprès du conducteur. Autrement dit, si l'unité de traitement 40 analyse que la décélération cardiaque débute tardivement, c'est-à-dire 3 secondes ou plus après la réception de l'indication D de danger, ou que la décélération cardiaque se termine plus de 3 secondes après cette réception d'indication D, elle commande au dispositif d'aide à la conduite 2 d'intervenir auprès du conducteur pour l'alerter et/ou l'aider.

L'analyse de la fréquence cardiaque à l'étape b) permet en outre à l'unité de traitement 40 de savoir si la perception du danger a beaucoup ou peu sollicité le conducteur. La sollicitation du conducteur est grande lorsque la durée de la décélération est longue et/ou lorsque la variation de fréquence cardiaque est grande, c'est-à-dire qu'elle descend bien au-dessous d'une valeur seuil prédéterminée. Grâce à l'analyse de la fréquence cardiaque à l'étape b), l'unité de traitement 40 peut donc déterminer si le danger a été perçu dans toute sa gravité ou non, ce qui permet à l'unité de traitement 40 d'affiner la commande du dispositif d'aide à la conduite 2.

Bien entendu, pour commander la ou les interventions du dispositif d'aide à la conduite, l'unité de traitement 40 est adaptée à tenir compte, en sus d'au moins un des éléments précités, de l'indication D du danger reçue à l'étape a), en particulier de la distance à laquelle se trouve le danger par rapport au véhicule, et de la dangerosité dudit danger.

L'unité de traitement 40 du dispositif 1 selon le troisième perfectionnement est ainsi adaptée à recevoir en entrée diverses informations relatives au danger et à la manière dont a été perçu ce danger par le conducteur, et à émettre, en sortie, une commande qui indique quelle doit être l'intervention du dispositif de conduite 2 auprès du conducteur. L'unité de traitement 40 garantit donc que la ou les interventions du dispositif d'aide à la conduite 2 sont émises au moment opportun, sous la forme la plus adaptée pour permettre l'adoption d'une réaction comportementale adaptée du conducteur.

Selon un quatrième perfectionnement avantageux du dispositif 1 selon l'invention, le dispositif 1 est capable d'identifier le conducteur pour proposer une analyse personnalisée de la fréquence cardiaque à l'étape b), et/ou une commande personnalisée du dispositif de conduite 2 à l'étape d).

Plus précisément, le dispositif 1 selon l'invention comprend un système d'identification 50, par exemple un système de reconnaissance faciale, ou de reconnaissance d'empreinte digitale. L'unité de traitement 40 est adaptée à communiquer avec ce système d'identification 50, de manière à identifier le conducteur et à récupérer des informations qu'elle a en mémoire sur ce conducteur.

L'unité de traitement 40 est alors adaptée à personnaliser, en fonction de ladite identification, l'analyse de la fréquence cardiaque mise en œuvre à l'étape b) et/ou la commande de l'étape d).

Grâce au système d'identification 50, les interventions du dispositif d'aide à la conduite 2 sont mieux adaptées au conducteur puisqu'il est possible de différencier un conducteur novice d'un conducteur expérimenté. En outre, selon la sensibilité du conducteur à certaines modalités d'alertes A plutôt qu'à d'autres, le dispositif d'aide à la conduite 2 privilégie l'émission d'alertes A avec les modalités préférées. Par exemple le dispositif d'aide à la conduite n'émet pas d'alertes visuelles à destination d'un conducteur n'ayant pas une bonne vue de près. Il émet à destination d'une personne âgée des alertes à modalités combinées, avec une certaine insistance, tandis qu'il émet à destination d'un conducteur expérimenté des alertes à une seule modalité.

Selon le même principe, grâce au système d'identification 50, les conseils C émis par le dispositif d'aide à la conduite 2 sont plus ou moins directifs, et leur modalité d'émission est aussi adaptée au conducteur. Les aides à la conduite I sont aussi déclenchées plus ou moins rapidement selon le conducteur au volant.

En outre, grâce au système d'identification 50, les paramètres qui définissent la décélération cardiaque (valeur seuil et durée) sont adaptés en fonction du conducteur identifié, de manière à augmenter la précision de l'analyse de l'étape b) par l'unité de traitement 40.

Ainsi, grâce au dispositif 1 selon l'invention, avec ou sans perfectionnement, on sait si le conducteur a correctement perçu un danger, de sorte qu'il est possible de concevoir des solutions de sécurité adaptées. En outre, grâce au dispositif selon l'invention, le dispositif d'aide à la conduite 2 intervient auprès du conducteur en fonction du danger mais aussi en fonction du conducteur au volant et des réactions cardiaques de ce conducteur, de sorte que l'habitacle n'est pas pollué par des alertes ou conseils intempestifs ou inadaptées à la situation et au conducteur. Le dispositif d'aide à la conduite 2 est ainsi mieux toléré par le conducteur.

L'invention se rapporte aussi à un procédé d'analyse de la perception d'un danger par un conducteur de véhicule tel que représenté sur la figure 4.

Selon le procédé de l'invention, il est prévu de mettre en œuvre les étapes de :

- mesure de la fréquence cardiaque FC du conducteur (représentée par le bloc E1 de la figure 4),
- réception de l'indication D du danger présent dans l'environnement du véhicule (représentée par le bloc E2 de la figure 4),
- analyse de la fréquence cardiaque FC du conducteur mesurée, dans l'intervalle de temps débutant à la réception de l'indication D (représentée par le bloc E3 de la figure 4), et
- détermination de la perception ou non du danger par le conducteur selon que la fréquence cardiaque du conducteur analysée comprend ou non une phase de décélération dans ledit intervalle de temps (représentée par le bloc E4 de la figure 4).

Le dispositif 1 selon l'invention est par exemple adapté à mettre en œuvre le procédé selon l'invention. L'étape de réception de l'indication D correspond à l'étape a) décrite précédemment, l'étape d'analyse de la fréquence cardiaque du conducteur correspond à l'étape b) décrite précédemment, et l'étape de détermination de la perception ou non du danger par le conducteur correspond à l'étape c) précédemment décrite.

Comme le montre la figure 4, à réception de l'indication D (bloc E2 de la figure 4) par l'unité de traitement 40 du dispositif 1, les mesures de fréquence cardiaque instantanées FC(t) du conducteur effectuées en continu sur le conducteur (bloc E1 de la figure 4) sont récupérées pour mettre en œuvre l'analyse de la fréquence cardiaque du conducteur (bloc E3 de la figure 4). Pour ce faire, l'unité de traitement 40 fixe l'instant de réception de l'indication D comme l'instant initial t=0s, et calcule la variation de fréquence cardiaque ΔFC(t) à partir de la fréquence cardiaque instantanée FC(t) du conducteur et de la fréquence de référence FCréf. En fonction de cette analyse, l'unité de traitement 40 détermine si le conducteur a perçu le danger ou non (bloc E4 de la figure 4).

Une fois cette détermination faite, l'unité de traitement 40 peut, de manière optionnelle (ce qui est représentée par les traits pointillés sur la figure 4), mettre en œuvre l'étape d) décrites précédemment, représentée par les blocs E5 sur la figure 4. Ainsi, l'unité de traitement 40 peut éventuellement commander les interventions du dispositif d'aide à la conduite 2 auprès du conducteur (bloc E5 de la figure 4). Cette étape optionnelle (bloc E5) correspond à l'étape d) décrite précédemment. La commande des interventions A, C, I par l'unité de traitement 40 comprend aussi bien le fait d'émettre les alertes A et/ou conseils C, que le fait de ne pas émettre lesdites alertes A et/ou conseils (par exemple en les mettant sous silence), ou encore le fait d'imposer ou non une aide à la conduite I au conducteur. Cette étape facultative (bloc E5) est mise en œuvre aussi bien si l'unité de traitement 40 conclut à l'étape de détermination (bloc E4) que le conducteur a perçu le danger (OUI), que si elle conclut qu'il n'a pas perçu le danger (NON).

Bien entendu, diverses autres modifications peuvent être apportées à l'invention dans le cadre des revendications annexées.

**Revendications**

1.  Dispositif (1) d'analyse de la perception d'un danger par un conducteur de véhicule, qui comprend :

    - un capteur de fréquence cardiaque (10) adapté à mesurer la fréquence cardiaque (FC) du conducteur au cours du temps (t), et,
    - une unité de traitement (40) adaptée à
    a) recevoir une indication (D) du danger présent dans l'environnement du véhicule,
    b) analyser la fréquence cardiaque (FC) du conducteur mesurée par le capteur de fréquence (10), dans un intervalle de temps débutant à la réception de l'indication (D) par l'unité de traitement, et
    c) déterminer si le danger a été perçu ou non par le conducteur selon que la fréquence cardiaque du conducteur analysée à l'étape b) comprend ou non une phase de décélération dans ledit intervalle de temps.

2.  Dispositif (1) selon la revendication 1, dans lequel à l'étape b), la fréquence cardiaque (FC) analysée est celle de l'intervalle de temps de mesure présentant une durée comprise entre 1 seconde et 5 secondes, à compter de la réception de l'indication (D).

3.  Dispositif (1) selon l'une quelconque des revendications 1 et 2, qui comprend en outre un capteur oculaire (30) adapté à détecter l'orientation du regard du conducteur, et dans lequel l'unité de traitement (40) détermine à l'étape c) que le danger a été perçu par le conducteur si la fréquence cardiaque du conducteur analysée à l'étape b) comprend une phase de décélération et si, en outre, le regard du conducteur est orienté dans le champ de conduite.

4.  Dispositif (1) selon l'une quelconque des revendications 1 à 3, qui comprend en outre un capteur de la respiration (20) du conducteur adapté à mesurer le rythme respiratoire du conducteur, et dans lequel l'unité de traitement (40) est adaptée à corriger la fréquence cardiaque du conducteur analysée à l'étape b) en fonction dudit rythme respiratoire mesuré.

5. Dispositif (1) selon l'une quelconque des revendications 1 à 4, dans lequel l'unité de traitement (40) détermine que la fréquence cardiaque du conducteur comprend une phase de décélération lorsque l'analyse de la fréquence cardiaque à l'étape b) montre que la variation de fréquence cardiaque ($\Delta$FC) du conducteur à un instant t présente une valeur inférieure à la moyenne des valeurs prises par la variation de fréquence cardiaque ($\Delta$FC) aux deux instants précédents l'instant t.

6. Dispositif selon la revendication 5, dans lequel la variation de fréquence cardiaque ($\Delta$FC) du conducteur est calculée à partir d'une fréquence cardiaque de référence (FCréf), choisie parmi : la fréquence cardiaque du conducteur mesurée par le capteur de fréquence cardiaque à l'instant où l'indication est reçue par l'unité de traitement à l'étape a) et une fréquence cardiaque moyenne du conducteur mesurée par le capteur de fréquence cardiaque.

7. Dispositif (1) selon l'une quelconque des revendications 1 à 6, dans lequel le capteur de fréquence cardiaque (10) comprend un capteur de l'activité électrique du cœur ou un capteur de l'activité mécanique du cœur, et dans lequel ledit capteur de fréquence cardiaque (10) est disposé sur le volant, et/ou sur le siège du conducteur et/ou sur la ceinture du conducteur et/ou à distance du conducteur.

8. Dispositif (1) selon l'une quelconque des revendications 1 à 7, dans lequel ladite unité de traitement (40) est adaptée à :
d) commander les interventions d'un dispositif d'aide à la conduite (2) embarqué dans le véhicule auprès du conducteur, en fonction d'au moins un des éléments suivants : la perception ou non du danger par le conducteur déterminée à l'étape c), le moment de perception du danger par le conducteur, la durée de l'éventuelle phase de décélération de la fréquence cardiaque analysée à l'étape b), l'amplitude de l'éventuelle phase de décélération de la fréquence cardiaque analysée à l'étape b).

9. Dispositif (1) selon la revendication 8, qui comprend le dispositif d'aide à la conduite (2), et dans lequel l'indication reçue par ladite unité de traitement à l'étape a) est émise par ledit dispositif d'aide à la conduite (2).

10. Dispositif (1) selon l'une quelconque des revendications 8 et 9, dans lequel l'unité de traitement (40) est en outre adaptée à identifier le conducteur et à personnaliser, en fonction de ladite identification, l'analyse de la fréquence cardiaque mise en œuvre à l'étape b).

11. Dispositif (1) selon la revendication 10, dans lequel l'unité de traitement (40) est en outre adaptée à personnaliser la commande de l'étape d) en fonction de ladite identification.

12. Procédé d'analyse de la perception d'un danger par un conducteur de véhicule, selon lequel il est prévu de mettre en œuvre les étapes de :

- mesure de la fréquence cardiaque (FC) du conducteur,
- réception d'une indication (D) du danger présent dans l'environnement du véhicule,
- analyse de la fréquence cardiaque (FC) du conducteur mesurée, dans un intervalle de temps débutant à la réception de l'indication (D) du danger, et
- détermination de la perception ou non du danger par le conducteur selon que la fréquence cardiaque du conducteur analysée comprend ou non une phase de décélération dans ledit intervalle de temps.

**Patentansprüche**

1. Vorrichtung (1) zur Analyse der Wahrnehmung einer Gefahr durch einen Fahrer eines Fahrzeugs, die umfasst:

- einen Herzfrequenzsensor (10), der dazu geeignet ist, die Herzfrequenz (FC) des Fahrers im Laufe der Zeit (t) zu messen, und
- eine Verarbeitungseinheit (40), die dazu geeignet ist,
a) eine Angabe (D) der im Umfeld des Fahrzeugs vorhandenen Gefahr zu empfangen,
b) die von dem Frequenzsensor (10) gemessene Herzfrequenz (FC) des Fahrers in einem Zeitintervall zu analysieren, das bei dem Empfangen der Angabe (D) durch die Verarbeitungseinheit beginnt, und
c) zu bestimmen, ob die Gefahr von dem Fahrer wahrgenommen worden ist oder nicht, je nachdem, ob die im Schritt b) analysierte Herzfrequenz des Fahrers eine Verlangsamungsphase in dem Zeitintervall umfasst oder nicht.

2. Vorrichtung (1) nach Anspruch 1, wobei im Schritt b) die analysierte Herzfrequenz (FC) diejenige des Messzeitintervalls ist, das eine Dauer zwischen 1 Sekunde und 5 Sekunden ab dem Empfangen der

**11**

Angabe (D) aufweist.

3. Vorrichtung (1) nach einem der Ansprüche 1 und 2, die ferner einen Augensensor (30) umfasst, der dazu geeignet ist, die Richtung des Blicks des Fahrers zu erkennen, und bei der die Verarbeitungseinheit (40) im Schritt c) bestimmt, dass die Gefahr von dem Fahrer wahrgenommen worden ist, wenn die im Schritt b) analysierte Herzfrequenz des Fahrers eine Verlangsamungsphase umfasst und wenn, ferner, der Blick des Fahrers in das Fahrfeld gerichtet ist.

4. Vorrichtung (1) nach einem der Ansprüche 1 bis 3, die ferner einen Sensor für die Atmung (20) des Fahrers umfasst, der dazu geeignet ist, den Atemrhythmus des Fahrers zu messen, und bei der die Verarbeitungseinheit (40) dazu geeignet ist, die im Schritt b) analysierte Herzfrequenz des Fahrers in Abhängigkeit von dem gemessenen Atemrhythmus zu korrigieren.

5. Vorrichtung (1) nach einem der Ansprüche 1 bis 4, wobei die Verarbeitungseinheit (40) bestimmt, dass die Herzfrequenz des Fahrers eine Verlangsamungsphase umfasst, wenn die Analyse der Herzfrequenz im Schritt b) ergibt, dass die Herzfrequenzänderung ($\Delta$FC) des Fahrers zu einem Zeitpunkt t einen Wert aufweist, der geringer als der Mittelwert der Werte ist, die von der Herzfrequenzänderung ($\Delta$FC) zu den zwei vorhergehenden Zeitpunkten t angenommen wurden.

6. Vorrichtung nach Anspruch 5, bei der die Herzfrequenzänderung ($\Delta$FC) des Fahrers ausgehend von einer Referenzherzfrequenz (FCréf) berechnet wird, die ausgewählt ist unter: der Herzfrequenz des Fahrers, die von dem Herzfrequenzsensor zu dem Zeitpunkt gemessen wird, zu dem die Angabe von der Verarbeitungseinheit im Schritt a) empfangen wird, und einer durchschnittlichen Herzfrequenz des Fahrers, die von dem Herzfrequenzsensor gemessen wird.

7. Vorrichtung (1) nach einem der Ansprüche 1 bis 6, bei welcher der Herzfrequenzsensor (10) einen Sensor für die elektrische Aktivität des Herzens oder einen Sensor für die mechanische Aktivität des Herzens umfasst und bei welcher der Herzfrequenzsensor (10) an dem Lenkrad und/oder an dem Sitz des Fahrers und/oder an dem Gurt des Fahrers und/oder von dem Fahrer beabstandet angeordnet ist.

8. Vorrichtung (1) nach einem der Ansprüche 1 bis 7, wobei die Verarbeitungseinheit (40) dazu geeignet ist:
d) Eingriffe einer Fahrassistenzvorrichtung (2), die bei dem Fahrer in das Fahrzeug integriert ist, in Abhängigkeit von mindestens einem der folgenden Umstände zu steuern: der im Schritt c) bestimmten Wahrnehmung oder Nichtwahrnehmung der Gefahr durch den Fahrer, dem Zeitpunkt der Wahrnehmung der Gefahr durch den Fahrer, der Dauer der etwaigen Verlangsamungsphase der im Schritt b) analysierten Herzfrequenz, der Amplitude der etwaigen Verlangsamungsphase der im Schritt b) analysierten Herzfrequenz.

9. Vorrichtung (1) nach Anspruch 8, welche die Fahrassistenzvorrichtung (2) umfasst und bei der die von der Verarbeitungseinheit im Schritt a) empfangene Angabe von der Fahrassistenzvorrichtung (2) gesendet wird.

10. Vorrichtung (1) nach einem der Ansprüche 8 und 9, bei der die Verarbeitungseinheit (40) ferner dazu geeignet ist, den Fahrer zu identifizieren und, in Abhängigkeit von der Identifizierung, die im Schritt b) durchgeführte Analyse der Herzfrequenz zu personalisieren.

11. Vorrichtung (1) nach Anspruch 10, bei der die Verarbeitungseinheit (40) ferner dazu geeignet ist, die Steuerung des Schritts d) in Abhängigkeit von der Identifizierung zu personalisieren.

12. Verfahren zur Analyse der Wahrnehmung einer Gefahr durch einen Fahrer eines Fahrzeugs, bei dem vorgesehen ist, die folgenden Schritte durchzuführen:

   - Messen der Herzfrequenz (FC) des Fahrers,
   a) Empfangen einer Angabe (D) der im Umfeld des Fahrzeugs vorhandenen Gefahr,
   b) Analysieren der gemessenen Herzfrequenz (FC) des Fahrers in einem Zeitintervall, das bei dem Empfangen der Angabe (D) der Gefahr beginnt, und
   c) Bestimmen der Wahrnehmung oder Nichtwahrnehmung der Gefahr durch den Fahrer, je nachdem, ob die analysierte Herzfrequenz des Fahrers eine Verlangsamungsphase in dem Zeitintervall umfasst oder nicht.

**Claims**

1. Device (1) for analysing a vehicle driver's perception of a danger, which device comprises:

   - a heart rate sensor (10) designed to measure the driver's heart rate (FC) over time (t), and
   - a processing unit (40) designed to
   a) receive an indication (D) of the danger present in the surroundings of the vehicle,
   b) analyse the driver's heart rate (FC) that was measured by the heart rate sensor (10), for a time interval which starts upon reception of the indication (D) by the processing unit, and
c) determine if the danger has been perceived or not by the driver depending on whether the driver's heart rate that was analysed in step b) comprises or does not comprise a deceleration phase within said time interval.

2. Device (1) according to Claim 1, wherein, in step b), the heart rate (FC) analysed is that of the time interval in which the measurement is taken, having a duration of between 1 second and 5 seconds, starting from the reception of the indication (D).

3. Device (1) according to either one of Claims 1 and 2, which moreover comprises an eye sensor (30) designed to detect the direction in which the driver is looking, and wherein the processing unit (40) determines in step c) that the danger has been perceived by the driver if the driver's heart rate that was analysed in step b) comprises a deceleration phase and if, furthermore, the driver is looking at the field of driving.

4. Device (1) according to any one of Claims 1 to 3, which moreover comprises a respiration sensor (20) for sensing the driver's breathing that is designed to measure the driver's respiratory rate, and wherein the processing unit (40) is designed to correct the driver's heart rate that was analysed in step b) depending on said measured respiratory rate.

5. Device (1) according to any one of Claims 1 to 4, wherein the processing unit (40) determines that the driver's heart rate comprises a deceleration phase when the analysis of the heart rate in step b) shows that the variation in the driver's heart rate ($\Delta$FC) at an instant t has a lower value than the mean of the values adopted by the variation in heart rate ($\Delta$FC) at the two instants preceding the instant t.

6. Device according to Claim 5, wherein the variation in the driver's heart rate ($\Delta$FC) is calculated from a reference heart rate (FCréf), selected from: the driver's heart rate measured by the heart rate sensor at the instant when the indication is received by the processing unit in step a) and the driver's mean heart rate measured by the heart rate sensor.

7. Device (1) according to any one of Claims 1 to 6, wherein the heart rate sensor (10) comprises a sensor for sensing the electrical activity of the heart or a sensor for sensing the mechanical activity of the heart, and wherein said heart rate sensor (10) is disposed on the steering wheel, and/or on the driver's seat and/or on the driver's seatbelt and/or remote from the driver.

8. Device (1) according to any one of Claims 1 to 7, wherein said processing unit (40) is designed to:
d) command a driving assistance device (2) installed on board the vehicle to intervene with the driver, depending on at least one of the following elements: the driver's perception or lack of perception of the danger determined in step c), the moment the danger was perceived by the driver, the duration of the possible phase of deceleration of the heart rate analysed in step b), the amplitude of the possible phase of deceleration of the heart rate analysed in step b).

9. Device (1) according to Claim 8, which comprises the driving assistance device (2), and wherein the indication received by said processing unit in step a) is sent by said driving assistance device (2).

10. Device (1) according to either one of Claims 8 and 9, wherein the processing unit (40) is moreover designed to identify the driver and to customize, depending on said identification,
the analysis of the heart rate implemented in step b).

11. Device (1) according to Claim 10, wherein the processing unit (40) is moreover designed to customize the command in step d) depending on said identification.

12. Method for analysing a vehicle driver's perception of a danger, according to which provision is made to implement the following steps:

- measuring the heart rate (FC) of the driver,
- receiving an indication (D) of the danger present in the surroundings of the vehicle,
- analysing the driver's heart rate (FC) that was measured, for a time interval which starts upon reception of the indication (D) of the danger, and
- determining if the danger has been perceived or not by the driver depending on whether the driver's heart rate that was analysed comprises or does not comprise a deceleration phase within said time interval.

# Fig.1

# Fig.2

# Fig.3

# Fig.4

# EP 4 271 601 B1

**Documents brevets cités dans la description**

- EP 1182089 A2
- US 2007146146 A1
- DE 102019201939 A1